# EUROPEAN PATENT SPECIFICATION

(11) **EP 1 414 431 B1**
(45) Date of publication and mention of the grant of the patent: **16.07.2008**
(21) Application number: 02735772.2
(22) Date of filing: 10.06.2002
(51) Int. Cl.: A61K 31/198, A61K 31/401, A61P 17/02

(54) **AMINO-ACID-BASED therapeutic COMPOSITIONS, FOR THE HEALING AND/OR MENDING OF WOUNDS AND LESIONS, IN PARTICULAR IN THE OPHTHALMIC FIELD**
AMINOSÄUREN-ENTHALTENDE ZUSAMMENSETZUNGEN, GEEIGNET ZUR WUNDHEILUNG, INSBESONDERE ZUR BEHANDLUNG DES AUGES
COMPOSITIONS thérapeutiques A BASE D'ACIDES AMINES POUR GUERIR ET/OU PANSER LES PLAIES ET LES LESIONS, NOTAMMENT DANS LE DOMAINE DE L'OPHTALMOLOGIE

(30) Priority: 08.08.2001 IT TO20010080
(43) Date of publication of application: 06.05.2004
(73) Proprietor: Solartium Enterprises Limited, Dublin 6 (IE)
(72) Inventor: CONTI, Franco, I-20121 Milano (IT); DIOGUARDI, Francesco, Saverio, I-20122 Milano (IT)
(74) Representative: Gallarotti, Franco
(86) International application number: PCT/IB2002/002147
(87) International publication number: WO 2003/013487

(56) References cited:
- EP-A- 0 034 504
- EP-A- 1 010 427
- WO-A-82/00411
- FR-A- 2 317 917
- GB-A- 1 050 756
- JP-A- 59 137 422
- US-A- 4 279 917
- US-A- 5 198 465
- DATABASE WPI Section Ch, Week 198437 Derwent Publications Ltd., London, GB; Class B04, AN 1984-229082 XP002232708 & JP 59 137422 A (KENKO IGAKUSHA KK), 7 August 1984 (1984-08-07)
- JAIS ABDUL MANAN MAT ET AL: "Fatty Acid and Amino Acid Composition in Haruan as a Potential Role in Wound Healing." GENERAL PHARMACOLOGY, vol. 25, no. 5, 1994, pages 947-950, XP009006260 ISSN: 0306-3623

## Description

The present invention relates to amino-acid-based compositions, suitable in therapy for the healing and/or mending of wounds and lesions, in particular for application in the ophthalmic field.

Amino acid compositions and solutions according to the preamble of claim 1 are known from GB-A-1 050 756, EP-A-0 034 504 and JP-A-59 137422+Database WPI, Derwent Publication Ltd, AN 1984-229082.

A composition according to the preamble of claim 1 is also present in the fish *Haruan C. Striatus,* which is consumed by Malaysians to induce healing of wounds (see "Fatty acid and amino acid Composition in Haruan as a potential role in wound healing", Jais Abdul Mana et al., General Pharm. Vol. 25, N. 5, 1994, pages 947-950).

From US-A-5,198,465 a composition is also known, with a base of proline, glycine and lysine, possibly comprising also methionine, cystine, cysteine, α-ketoglutaric acid and vitamin C, the said composition being able to induce or promote biological synthesis of collagen in situations in which said synthesis is lacking.

Starting from the above known technique, the purpose of the present invention is to indicate new amino-acid-based compositions which will prove particularly effective in therapy for healing and/or mending wounds and lesions, in particular for application in the ophthalmic field.

In this perspective, the inventors have succeeded in obtaining the formulation of amino-acid-based compositions in accordance with claim 1.

The compositions according to the invention may comprise one or more additional amino acids, with respect to the ones mentioned in claim 1, the sum of which, expressed in molecular weights, is preferably of a percentage smaller than 20% with respect to the sum of the other active ingredients, and less than 10% for each individual additional amino acid.

The application further refers to the following.

Composition according to Claim 1, **characterized in that** the sum of the amounts, expressed in molecular weight, of threonine and lysine
- is smaller than the sum of the individual amounts of glycine and proline, and/or
- is smaller than the sum of the individual amounts of leucine, isoleucine and valine.

Composition according to Claim 1, **characterized in that** the sum of leucine, isoleucine, valine and threonine is up to 75 wt% of the total of all the amino acids or active ingredients envisaged.

Composition according to Claim 1, **characterized in that** it comprises at least one of methionine and tyrosine as further active ingredient.

Composition according to any one of the preceding claims, **characterized in that** it comprises cyst(e)ine, i.e., cystine and cysteine, as further active ingredient, preferably in a molar ratio with methionine equal to or higher than 2:1.

Composition according to Claim 5, **characterized in that** they comprise one or more additional amino acids, the sum of which, expressed in molecular weight, is in a percentage lower than 20% with respect to the sum of the other active ingredients and less than 10% for each individual additional amino acid.

Composition according to at least one of the preceding claims, **characterized in that** they comprise:
- between 8 wt% and 40 wt% of glycine on the total of the amino acids envisaged;
- between 7 wt% and 40 wt% of proline on the total of the amino acids envisaged;
- between 3 wt% and 35 wt% of lysine on the total of the amino acids envisaged;
the sum of glycine, proline and lysine being, in particular, not less than 18 wt% of the total of the amino acids envisaged.

Composition according to at least one of the preceding claims, **characterized** they comprise:
- between 4 wt% and 40 wt% of leucine on the total of the amino acids envisaged;
- between 2 wt% and 20 wt% of isoleucine on the total of the amino acids envisaged;
- between 2 wt% and 20 wt% of valine on the total of the amino acids envisaged;
- up to 20 wt% of threonine on the total of the amino acids envisaged.

Composition according to the preceding claim, **characterized in that** the sum of leucine, isoleucine, valine and threonine is between 10 wt% and 50 wt% on the total of the amino acids envisaged.

Composition according to Claim 8, **characterized in that** leucine, isoleucine and valine are in a stoichiometric ratio of 2:1:1.

Composition according to at least one of the preceding claims, **characterized in that** threonine plus lysine are in a molar ratio with respect to one another with leucine, isoleucine and valine of between 20% and 70%, preferably with a ratio between threonine and lysine in which lysine is more represented than threonine.

Composition according to at least one of the preceding claims, **characterized in that** histidine is present in a molar ratio of up to 50% of the following amino acids:
- cyst(e)ine (i.e., cystine and cysteine) and methionine;
- phenyl alanine and tyrosine;
- tryptophan, in particular in a molar ratio of up to 5% of the weight of all the other amino acids on a basis of molar weight.

Process for the preparation of amino-acid-based composition, suitable in therapy for the healing and/or mending of wounds and lesions, in particular for application in the ophthalmic field, providing for the use of
- proline, glycine and lysine, up to 80 wt% on the total of all the amino acids or active ingredients envisaged,
- leucine, isoleucine and threonine, in an overall quantity of between 2 wt% and 60 wt% on the total of all the amino acids or active ingredients envisaged,
- valine,
- one or more of the other essential amino-acids phenyl alanine, histidine, tryptophan and methionine,
**characterized in that**
- the amounts, expressed in molecular weight, of threonine and lysine are each greater than the sum of the individual amounts of the said other essential amino acids present in the composition, but:
   - the amount of threonine is smaller than the individual amounts of glycine, proline, lysine, leucine, isoleucine and valine;
   - the amount of lysine is smaller than the individual amounts of glycine, proline and leucine.

Process according to Claim 13, **characterized in that** the sum of the amounts, expressed in molecular weight, of threonine and lysine
- is smaller than the sum of the individual amounts of glycine and proline, and/or
- is smaller than the sum of the individual amounts of leucine, isoleucine and valine.

Process according to Claim 13, **characterized by** the use of at least one of methionine and tyrosine as further active ingredient.

Process according to any one of the preceding claims, **characterized by** the use of cyst(e)ine, i.e., cystine and cysteine, as further active ingredient, preferably in a molar ratio with methionine equal to or higher than 2:1.

Process according to Claim 16, **characterized by** the use of one or more additional amino acids, the sum of which, expressed in molecular weight, is in a percentage lower than 20% with respect to the sum of the other active ingredients and less than 10% for each individual additional amino acid.

Use of the amino-acids proline, glycine and lysine in combination with
- leucine, isoleucine and threonine,
- valine, and
- one or more of the other essential aminoacids phenylalanine, histidine, tryptophan and methionine,
as active ingredients for the manufacture of composition for the healing and/or mending of wounds and lesions of the structural and stromal collagen,
proline, glycine and lysine being in a total amount of up to 80 wt% on the total of all the amino acids or active ingredients envisaged, and leucine, isoleucine and threonine being in an overall quantity of between 2 wt% and 60 wt% on the total of all the amino acids or active ingredients envisaged,
wherein the amounts, expressed in molecular weight, of threonine and lysine are each greater than the sum of the individual amounts of the said other essential amino acids present in the composition, but:
- the amount of threonine is smaller than the individual amounts of glycine, proline, lysine, leucine, isoleucine and valine;
- the amount of lysine is smaller than the individual amounts of glycine, proline and leucine.

Use according to Claim 18, wherein the sum of the amounts, expressed in molecular weight, of threonine and lysine
- is smaller than the sum of the individual amounts of glycine and proline, and/or
- is smaller than the sum of the individual amounts of leucine, isoleucine and valine.

Use according to Claim 18, wherein at least one of methionine and tyrosine is provided as further active ingredient.

Use according to any one of the preceding claims, wherein cyst(e)ine, i.e., cystine and cysteine, is provided as further active ingredient, preferably in a molar ratio with methionine equal to or higher than 2:1.

Use according to Claim 21, wherein one or more additional amino acids are provided, the sum of which, expressed in molecular weight, is in a percentage lower than 20% with respect to the sum of the other active ingredients and less than 10% for each individual additional amino acid.

The preferred formulations of the compositions according to the invention, comprising essential and non-essential amino acids (glycine, proline, lysine, leucine, isoleucine, valine, threonine, methionine, phenyl alanine, histidine, tryptophan, tyrosine and cyst(e)ine) fall within the following spheres (in what follows, where not otherwise specified, the weight percentages of the various amino acids on the total thereof are indicated):
- glycine (8-40 wt%), proline (7-40 wt%), lysine (3-35 wt%), which account for 18-80 wt% of the entire composition of amino acids;
- leucine (4-40 wt%), isoleucine (2-20 wt%), valine (2-20 wt%), threonine (up to 20 wt%), which account for 8-70 wt% of the entire composition of amino acids, where leucine, isoleucine and valine are preferably in a stoichiometric ratio 2:1:1 and where threonine plus lysine are preferably in a molar ratio with respect to one another with leucine, isoleucine and valine of between 20 and 70%, preferably with a ratio between threonine and lysine in which lysine is more represented than threonine; and
- histidine, present in molar fractions of up to 50% of the following amino acids:
   - cyst(e)ine (i.e., cystine and cysteine) and methionine, up to 50% of the histidine, where the ratio between cyst(e)ine and methionine should preferably be between 50 and 200% greater than the cyst(e)ine, in molar ratio;
   - phenyl alanine and tyrosine, in a molar ratio of up to 60% of the histidine (where the tyrosine is preferably represented by up to 50% of the molar weight of the phenyl aldnine);
   - tryptophan up to 5% of the weight of all the other amino acids on a basis of molar weight.

As has been said, any other amino acid can be added to the aforesaid formulation without altering the expected effects thereof, provided that the sum of the additional amino acids is in a percentage lower than 20 wt% with respect to the sum of the other active ingredients (less than 10 wt% for each amino acid).

With the aim of demonstrating the effectiveness of the mixture according to the invention for the purposes of therapy for healing wounds and lesions, experimental tests have been carried out, aimed at testing the stimulating action on the cell-proliferation activity of two stocks of cells (corneal fibroblasts and conjunctival cells) performed by two mixtures of amino acids.

The first mixture was obtained according to the teachings of US-A-5,198,465 and contained only glycine, proline, lysine and vitamin C.

The second mixture, obtained according to the present invention, had the following composition:

| **Amino acids** | **Amounts in mg** (per g. of mixture) | **Weight percent** (on the total of amino acids) |
|---|---|---|
| Glycine | 250,0 | 25.00% |
| Proline | 218,8 | 21.88% |
| Lysine | 112,5 | 11.25% |
| Leucine | 156,3 | 15.63% |
| Isoleucine | 78,1 | 7.81% |
| Valine | 78,1 | 7.81% |
| Threonine | 43,8 | 4.38% |
| Methionine | 6,3 | 0.63% |
| Phenyl alanine | 12,5 | 1.25% |
| Histidine | 18,8 | 1.88% |
| Tryptophan | 2,5 | 0.25% |
| Tyrosine | 3,8 | 0.38% |
| Cyst(e)ine | 18,8 | 1.88% |

The activity of the two mixtures subjected to comparative analysis was tested both *in vitro* and *in vivo*.

### In-vitro tests

The cell lines chosen for development of the experimental model were: rabbit corneal fibroblast cells (SIRC) and human conjunctival cells (1-5C-4). The cell lines used were exposed to a dose-response curve developed in a concentration range of from 0.1 mg/ml to 1 mg/ml of the two mixtures of amino acids, i.e., the mixture obtained according to the teachings of US-A-5,198,465 and the mixture obtained in accordance with the present invention.

The products were solubilized and then diluted at the experimental concentrations, using a culture medium without any serum component. The cell response to exposure was assessed using the MTT colorimetric test, a method which enables definition of the residual vitality of the cells exposed to the product, quantifying the metabolic functionality of the mitochondria. This evaluation was made on the 3^{rd}, 6^{th} and 8^{th} day.

For each experimental point of the dose-response curve, the cell response of 8 wells was evaluated. The values of absorbance were subjected to statistical analysis for determination of the mean value, the standard error and significance (Student's *t*).

In the tables given below, which summarize the data obtained from the individual recordings, the numerical values are expressed as percentage of cell vitality with respect to a control that had not been exposed to the product, and to which a vitality of 100% was attributed.

Tables 1 and 2 appearing below show, in particular, the dose-response curve in the absence of bovine foetal serum of fibroblasts, respectively for the mixture obtained according to the teachings of US-A-5 198 465 and for the mixture according to the invention.

**TABLE 1 (mixture according to US-A-5,198,465)**

| **mg/ml** | **3^{rd} day** | **6^{th} day** | **8^{th} day** |
|---|---|---|---|
| 0 | 100 | 100 | 100 |
| 0.1 | 126.02 * | 131.08 # | 206.6 # |
| 0.25 | 129.27 * | 148.09 # | 219.4 # |
| 0.5 | 127.78 * | 169.65 # | 235.41 # |
| 1 | 125 * | 153.75 # | 229 # |

| | | | |
|---|---|---|---|
| * = p<0.005 vs. control # = p<0.0001 vs. control | | | |

**TABLE 2 (mixture according to the invention)**

| **mg/ml** | **3^{rd} day** | **6^{th} day** | **8^{th} day** |
|---|---|---|---|
| 0 | 100 | 100 | 100 |
| 0.1 | 14Q.31 #° | 178.70 #°° | 220.53 #° |
| 0.25 | 154.01 #° | 180.42 #°° | 230.91 #° |
| 0.5 | 160.22 #° | 193.08 #°° | 260.07 #° |
| 1 | 163.04 #° | 190.23 #°° | 261.18 #°° |

| | | | |
|---|---|---|---|
| # = p<0.0001 vs. control ° = p<0.005 vs. US-A-5,198,465 mixture °° = p<0.0001 vs. US-A-5,198,465 mixture | | | |

As may be seen from Table 1, the mixture according to the known art expresses a stimulant activity on the proliferation of the fibroblasts that is already statistically significant on the third day for all the concentrations tested and becomes more evident on the sixth day and on the eighth day with increments that are approximately dose-dependent. From Table 2, it may be noted, instead, how the mixture according to the invention is able to produce a faster stimulation in time, with increments in comparison with the mixture according to the prior art that are already statistically significant on the third day. From a comparison between Tables 1 and 2, it clearly emerges how the mixture according to the invention is decidedly more effective on the proliferation of fibroblasts, which thus leads to a reduction in the response times and to a further increase in the number of cells.

Tables 3 and 4 appearing below show, instead, the dose-response curve in the absence of bovine foetal serum in the conjunctival-cell line, respectively for the mixture obtained according to the teachings of US-A-5,198,465 and for the mixture according to the invention.

**TABLE 3 (mixture according to US-A-5,198,465)**

| **mg/ml** | **3^{rd} day** | **6^{th} day** | **8^{th} day** |
|---|---|---|---|
| 0 | 100 | 100 | 100 |
| 0.1 | 91.14 | 102.32 | 109.71 |
| 0.25 | 104.3 | 107.91 | 108.08 |
| 0.5 | 111.06 | 109.84 | 110.13 |
| 1 | 101.02 | 113.06 * | 118.77 * |

| | | | |
|---|---|---|---|
| * = p<0.005 vs. control | | | |

**TABLE 4 (mixture according to the invention)**

| **mg/ml** | **3^{rd} day** | **6^{th} day** | **8^{th} day** |
|---|---|---|---|
| 0 | 100 | 100 | 100 |
| 0.1 | 87.7 | 108.16 *° | 138.97 #°° |
| 0.25 | 112.83 | 122.45 *° | 178.07 #°° |
| 0.5 | 100.97 | 133.13 *° | 193.81 #°° |
| 1 | 113.91 | 126.26 *°° | 201.68 #°° |

| | | | |
|---|---|---|---|
| * = p<0.005 vs. control # = p<0.0001 vs. control ° = p<0.005 vs. US-A-5,198,465 mixture °° = p<0.0001 vs. US-A-5,198,465 mixture | | | |

From Table 3, it may be noted how the conjunctival cells presented a poor response to the addition of the mixture according to the prior art in the culture medium, and only at the dose of 1 mg/ml at day 6 and day 8 showed a modest significant increase.

From Table 4, it may instead be noted how the mixture according to the invention did not reveal a significant increase at day 3, but at day 6 and day 8 the increments in cell proliferation were markedly evident and significant both in regard to the controls and in regard to the mixture according to the prior art.

From the above results, it is thus evident how the mixture according to the invention is able to stimulate the two fundamental cell stocks for repairing corneal lesions, ensuring a rapid formation of the corneal stroma, of the basal lamina, and hence a fast re-epithelization of the mucosae.

### In-vivo tests

To test the effectiveness of the mixture of amino acids according to the invention as compared to the mixture according to US-A-5,198,465, 20 patients affected by corneal ulcers for over 18 months and resistant to normal treatments were chosen, who had been observed for three months prior to start of treatment.

The group consisted of 12 men and 8 women with an average age of 58 years. Of these 6 were diabetics of type 2, who were being treated with drugs of a hypoglycaemic type and were in good metabolic compensation.

The cases were randomly divided into two groups of 10 patients each, with 3 diabetics in each group.

Throughout the experimental period, no drug was changed, either topical drug or drug to be administered via general route, and only the two mixtures according to the invention were added. The mixture according to the prior art was administered via oral route at the dosage of 12 g divided into three administrations per day. Also the mixture according to the invention was administered via oral route at the dosage of 12 g, once again divided into three administrations per day. Treatment lasted one month.

The evaluation of the therapeutic activity envisaged just two possibilities: complete healing of the lesion; and no healing within one month of treatment.

Of the 10 subjects treated with the mixture according to the invention, complete healing was found in 9 subjects, whereas using the mixture according to US-A-5,198,465, a case of healing was found in just one subject and improvements in a further four subjects (reduction in the diameter of the lesion).

From the above findings, it is thus noted how the treatment with the mixture according to the invention has proven itself to be clearly superior to treatment with the mixture according to US-A-5,198,465, with a healing of the lesion in 90% of the cases (p<0.05).

The compositions according to the invention may be employed for administration via oral route (pills, tablets, powders, etc.), for topical administration (collyrium, cream, gel, etc.), and for administration via parenteral route, for example via local injection. In connection with this latter possibility of use, an injectable aqueous solution may be envisaged, prepared extemporarily, dissolving the composition according to the invention, prepared previously in a lyophilized form, in a biologically compatible aqueous liquid (distilled water, physiological solution or other aqueous solution).

If so required, administration of the mixture may be in the form of a number of distinct preparations, for instance a tablet (or any other pharmaceutical formulation) containing some of the amino acids envisaged and/or fractions thereof (for example, glycine, proline, lysine), and a tablet (or any other pharmaceutical formulation) containing the other amino acids envisaged and/or fractions thereof (for example, leucine, isoleucine, threonine, and possibly lysine and/or methionine and/or phenyl alanine and/or histidine and/or tryptophan and/or tyrosine and/or cyst(e)ine).

Of course, for the purposes of preparation of the compositions according to the invention, it is possible to use diluents and excipients in any pharmacological form suited for the chosen use.

From the foregoing description, there emerge clearly the characteristics of the present invention, as likewise do the advantages afforded thereby, which are chiefly represented by the considerable efficacy in the therapy of healing and/or mending of wounds, lesions and ulcers, in particular by means of the increased proliferation of the cells of the stroma. The mixtures according to the present invention prove highly effective in the treatment of corneal ulcers and in the field of refractive surgery, but the sphere of application of the invention must not be understood as being limited to the area of ophthamology. In such a perspective, the invention must therefore be understood as extending to all those applications in which it is desirable to have a rapid healing or mending of lesions of any type, including bone fractures and traumas to internal organs.

The compositions according to the present invention may possibly envisage the addition of α-ketoglutaric acid, up to 20 wt% of the total weight, and vitamin C, between 10 wt% and 50 wt% of the total weight, the latter functioning, in particular, as co-enzyme of specific hydroxylase in the catalysis of the biological synthesis of collagen.

## Claims

1. Amino-acid-based composition, suitable in therapy for the healing and/or mending of wounds and lesions, in particular for application in the ophthalmic field, comprising:
- proline, glycine and lysine, in a total amount of up to 80 wt% on the total of all the amino acids or active ingredients envisaged,
- leucine, isoleucine and threonine, in an overall quantity of between 2 wt% and 60 wt% on the total of all the amino acids or active ingredients envisaged,
- valine,
- one or more of the other essential amino-acids phenyl alanine, histidine, tryptophan and methionine,
**characterized in that**
- the amounts, expressed in molecular weight, of threonine and lysine are each greater than the sum of the individual amounts of the said other essential amino acids present in the composition, but:
- the amount of threonine is smaller than the individual amounts of glycine, proline, lysine, leucine, isoleucine and valine;
- the amount of lysine is smaller than the individual amounts of glycine, proline and leucine.

2. Composition according to Claim 1, **characterized in that** the sum of the amounts, expressed in molecular weight, of threonine and lysine
- is smaller than the sum of the individual amounts of glycine and proline, and/or
- is smaller than the sum of the individual amounts of leucine, isoleucine and valine.

3. Composition according to Claim 1, **characterized in that** the sum of leucine, isoleucine, valine and threonine is up to 75 wt% of the total of all the amino acids or active ingredients envisaged.

4. Composition according to Claim 1, **characterized in that** it comprises at least one of methionine and tyrosine as further active ingredient.

5. Composition according to any one of the preceding claims, **characterized in that** it comprises cyst(e)ine, i.e., cystine and cysteine, as further active ingredient, preferably in a molar ratio with methionine equal to or higher than 2:1.

6. Composition according to Claim 5, **characterized in that** they comprise one or more additional amino acids, the sum of which, expressed in molecular weight, is in a percentage lower than 20% with respect to the sum of the other active ingredients and less than 10% for each individual additional amino acid.

7. Composition according to at least one of the preceding claims, **characterized in that** they comprise:
- between 8 wt% and 40 wt% of glycine on the total of the amino acids envisaged;
- between 7 wt% and 40 wt% of proline on the total of the amino acids envisaged;
- between 3 wt% and 35 wt% of lysine on the total of the amino acids envisaged;
the sum of glycine, proline and lysine being, in particular, not less than 18 wt% of the total of the amino acids envisaged.

8. Composition according to at least one of the preceding claims, **characterized** they comprise:
- between 4 wt% and 40 wt% of leucine on the total of the amino acids envisaged;
- between 2 wt% and 20 wt% of isoleucine on the total of the amino acids envisaged;
- between 2 wt% and 20 wt% of valine on the total of the amino acids envisaged;
- up to 20 wt% of threonine on the total of the amino acids envisaged.

9. Composition according to the preceding claim, **characterized in that** the sum of leucine, isoleucine, valine and threonine is between 10 wt% and 50 wt% on the total of the amino acids envisaged.

10. Composition according to Claim 8, **characterized in that** leucine, isoleucine and valine are in a stoichiometric ratio of 2:1:1.

11. Composition according to at least one of the preceding claims, **characterized in that** threonine plus lysine are in a molar ratio with respect to one another with leucine, isoleucine and valine of between 20% and 70%, preferably with a ratio between threonine and lysine in which lysine is more represented than threonine.

12. Composition according to at least one of the preceding claims, **characterized in that** histidine is present in a molar ratio of up to 50% of the following amino acids:
- cyst(e)ine (i.e., cystine and cysteine) and methionine;
- phenyl alanine and tyrosine;
- tryptophan, in particular in a molar ratio of up to 5% of the weight of all the other amino acids on a basis of molar weight.

13. Process for the preparation of amino-acid-based composition, suitable in therapy for the healing and/or mending of wounds and lesions, in particular for application in the ophthalmic field, providing for the use of
- proline, glycine and lysine, up to 80 wt% on the total of all the amino acids or active ingredients envisaged,
- leucine, isoleucine and threonine, in an overall quantity of between 2 wt% and 60 wt% on the total of all the amino acids or active ingredients envisaged,
- valine,
- one or more of the other essential amino-acids phenyl alanine, histidine, tryptophan and methionine,
**characterized in that**
- the amounts, expressed in molecular weight, of threonine and lysine are each greater than the sum of the individual amounts of the said other essential amino acids present in the composition, but:
- the amount of threonine is smaller than the individual amounts of glycine, proline, lysine, leucine, isoleucine and valine;
- the amount of lysine is smaller than the individual amounts of glycine, proline and leucine.

14. Process according to Claim 13, **characterized in that** the sum of the amounts, expressed in molecular weight, of threonine and lysine
- is smaller than the sum of the individual amounts of glycine and proline, and/or
- is smaller than the sum of the individual amounts of leucine, isoleucine and valine.

15. Process according to Claim 13, **characterized by** the use of at least one of methionine and tyrosine as further active ingredient.

16. Process according to any one of the preceding claims, **characterized by** the use of cyst(e)ine, i.e., cystine and cysteine, as further active ingredient, preferably in a molar ratio with methionine equal to or higher than 2:1.

17. Process according to Claim 16, **characterized by** the use of one or more additional amino acids, the sum of which, expressed in molecular weight, is in a percentage lower than 20% with respect to the sum of the other active ingredients and less than 10% for each individual additional amino acid.

18. Use of the amino-acids proline, glycine and lysine in combination with
- leucine, isoleucine and threonine,
- valine, and
- one or more of the other essential aminoacids phenylalanine, histidine, tryptophan and methionine,
as active ingredients for the manufacture of composition for the healing and/or mending of wounds and lesions of the structural and stromal collagen,
proline, glycine and lysine being in a total amount of up to 80 wt% on the total of all the amino acids or active ingredients envisaged, and leucine, isoleucine and threonine being in an overall quantity of between 2 wt% and 60 wt% on the total of all the amino acids or active ingredients envisaged,
wherein the amounts, expressed in molecular weight, of threonine and lysine are each greater than the sum of the individual amounts of the said other essential amino acids present in the composition, but:
- the amount of threonine is smaller than the individual amounts of glycine, proline, lysine, leucine, isoleucine and valine;
- the amount of lysine is smaller than the individual amounts of glycine, proline and leucine.

19. Use according to Claim 18, wherein the sum of the amounts, expressed in molecular weight, of threonine and lysine
- is smaller than the sum of the individual amounts of glycine and proline, and/or
- is smaller than the sum of the individual amounts of leucine, isoleucine and valine.

20. Use according to Claim 18, wherein at least one of methionine and tyrosine is provided as further active ingredient.

21. Use according to any one of the preceding claims, wherein cyst(e)ine, i.e., cystine and cysteine, is provided as further active ingredient, preferably in a molar ratio with methionine equal to or higher than 2:1.

22. Use according to Claim 21, wherein one or more additional amino acids are provided, the sum of which, expressed in molecular weight, is in a percentage lower than 20% with respect to the sum of the other active ingredients and less than 10% for each individual additional amino acid.

## Patentansprüche

1. Zusammensetzung auf Aminosäurebasis, die bei der Therapie zum Heilen und/oder Bessern von Wunden und Läsionen geeignet ist, insbesondere zur Anwendung auf ophthalmischem Gebiet, und enthält:
- Prolin, Glycin und Lysin in einer Gesamtmenge von bis zu 80 Gew.-% auf die Gesamtsumme aller ins Auge gefassten Aminosäuren oder Wirkstoffe,
- Leucin, Isoleucin und Threonin in einem Gesamtquantum von zwischen 2 Gew.-% und 60 Gew.-% auf die Gesamtsumme aller ins Auge gefassten Aminosäuren oder Wirkstoffe,
- Valin,
- eine oder mehr der anderen essentiellen Aminosäuren Phenylalanin, Histidin, Tryptophan und Methionin,
**dadurch gekennzeichnet, dass**
- die Mengen, ausgedrückt in Molekulargewicht, von Threonin und Lysin jeweils größer sind als die Summe der Einzelmengen der genannten anderen in der Zusammensetzung vorhandenen essentiellen Aminosäuren, aber:
- die Menge von Threonin kleiner ist als die Einzelmengen von Glycin, Prolin, Lysin, Leucin, Isoleucin und Valin;
- die Menge von Lysin kleiner ist als die Einzelmengen von Glycin, Prolin und Leucin.

2. Zusammensetzung gemäß Anspruch 1, **dadurch gekennzeichnet, dass** die Summe der Mengen, ausgedrückt in Molekulargewicht, von Threonin und Lysin
- kleiner ist als die Summe der Einzelmengen von Glycin und Prolin und/oder
- kleiner ist als die Summe der Einzelmengen von Leucin, lsoleucin und Valin.

3. Zusammensetzung gemäß Anspruch 1, **dadurch gekennzeichnet, dass** die Summe von Leucin, Isoleucin, Valin und Threonin bis zu 75 Gew.-% der Gesamtsumme aller ins Auge gefassten Aminosäuren oder Wirkstoffe ist.

4. Zusammensetzung gemäß Anspruch 1, **dadurch gekennzeichnet, dass** sie wenigstens eines von Methionin und Tyrosin als weiteren Wirkstoff enthält.

5. Zusammensetzung gemäß einem der vorhergehenden Ansprüche, **dadurch gekennzeichnet, dass** sie Cyst(e)in, d. h. Cystin und Cystein, als weiteren Wirkstoff enthält, vorzugsweise in einem molaren Verhältnis mit Methionin gleich wie oder höher als 2:1.

6. Zusammensetzung gemäß Anspruch 5, **dadurch gekennzeichnet, dass** sie eine oder mehr zusätzliche Aminosäuren enthält, deren Summe, ausgedrückt in Molekulargewicht, in einem Prozentsatz niedriger als 20 % in Bezug auf die Summe der anderen Wirkstoffe und weniger als 10 % für jede einzelne zusätzliche Aminosäure ist.

7. Zusammensetzung gemäß wenigstens einem der vorhergehenden Ansprüche,
**dadurch gekennzeichnet, dass** sie enthält:
- zwischen 8 Gew.-% und 40 Gew.-% Glycin auf die Gesamtsumme der ins Auge gefassten Aminosäuren;
- zwischen 7 Gew.-% und 40 Gew.-% Prolin auf die Gesamtsumme der ins Auge gefassten Aminosäuren;
- zwischen 3 Gew.-% und 35 Gew.-% Lysin auf die Gesamtsumme der ins Auge gefassten Aminosäuren;
wobei die Summe von Glycin, Prolin und Lysin insbesondere nicht weniger als 18 Gew.-% der Gesamtsumme der ins Auge gefassten Aminosäuren ist.

8. Zusammensetzung gemäß wenigstens einem der vorhergehenden Ansprüche,
**dadurch gekennzeichnet, dass** sie enthält:
- zwischen 4 Gew.-% und 40 Gew.-% Leucin auf die Gesamtsumme der ins Auge gefassten Aminosäuren;
- zwischen 2 Gew.-% und 20 Gew.-% Isoleucin auf die Gesamtsumme der ins Auge gefassten Aminosäuren;
- zwischen 2 Gew.-% und 20 Gew.-% Valin auf die Gesamtsumme der ins Auge gefassten Aminosäuren;
- bis zu 20 Gew.-% Threonin auf die Gesamtsumme der ins Auge gefassten Aminosäuren.

9. Zusammensetzung gemäß dem vorhergehenden Anspruch, **dadurch gekennzeichnet, dass** die Summe von Leucin, lsoleucin, Valin und Threonin zwischen 10 Gew.-% und 50 Gew.-% auf die Gesamtsumme der ins Auge gefassten Aminosäuren ist.

10. Zusammensetzung gemäß Anspruch 8, **dadurch gekennzeichnet, dass** Leucin, Isoleucin und Valin in einem stöchiometrischen Verhältnis von 2:1:1 stehen.

11. Zusammensetzung gemäß wenigstens einem der vorhergehenden Ansprüche, **dadurch gekennzeichnet, dass** Threonin plus Lysin mit Leucin, Isoleucin und Valin zwischen 20 % und 70 % in einem molaren Verhältnis in Bezug zueinander stehen, vorzugsweise mit einem Verhältnis zwischen Threonin und Lysin, in dem Lysin mehr vertreten ist als Threonin.

12. Zusammensetzung gemäß wenigstens einem der vorhergehenden Ansprüche,
**dadurch gekennzeichnet, dass** Histidin in einem molaren Verhältnis von bis zu 50 % der folgenden Aminosäuren vorhanden ist:
- Cyst(e)in (d. h. Cystin und Cystein) und Methionin;
- Phenylalanin und Tyrosin;
- Tryptophan, insbesondere in einem molaren Verhältnis von bis zu 5 % des Gewichts aller anderen Aminosäuren auf Molgewichtsbasis.

13. Verfahren für die Zubereitung einer Zusammensetzung auf Aminosäurebasis, die bei der Therapie zum Heilen und/oder Bessern von Wunden und Läsionen geeignet ist, insbesondere zur Anwendung auf ophthalmischem Gebiet, unter Vorsehen der Verwendung von:
- Prolin, Glycin und Lysin bis zu 80 Gew.-% auf die Gesamtsumme aller ins Auge gefassten Aminosäuren oder Wirkstoffe,
- Leucin, Isoleucin und Threonin in einem Gesamtquantum von zwischen 2 Gdw.-% und 60 Gew.-% auf die Gesamtsumme aller ins Auge gefassten Aminosäuren oder Wirkstoffe,
- Valin,
- einer oder mehr der anderen essentiellen Aminosäuren Phenylalanin, Histidin, Tryptophan und Methionin,
**dadurch gekennzeichnet, dass**
- die Mengen, ausgedrückt in Molekulargewicht, von Threonin und Lysin jeweils größer sind als die Summe der Einzelmengen der genannten anderen in der Zusammensetzung vorhandenen essentiellen Aminosäuren, aber:
- die Menge von Threonin kleiner ist als die Einzelmengen von Glycin, Prolin, Lysin, Leucin, Isoleucin und Valin;
- die Menge von Lysin kleiner ist als die Einzelmengen von Glycin, Prolin und Leucin.

14. Verfahren gemäß Anspruch 13, **dadurch gekennzeichnet, dass** die Summe der Mengen, ausgedrückt in Molekulargewicht, von Threonin und Lysin
- kleiner ist als die Summe der Einzelmengen von Glycin und Prolin und/oder
- kleiner ist als die Summe der Einzelmengen von Leucin, lsoleucin und Valin.

15. Verfahren gemäß Anspruch 13, **gekennzeichnet durch** die Verwendung wenigstens eines von Methionin und Tyrosin als weiteren Wirkstoff.

16. Verfahren gemäß einem der vorhergehenden Ansprüche, **gekennzeichnet durch** die Verwendung von Cyst(e)in, d. h. Cystin und Cystein, als weiteren Wirkstoff, vorzugsweise in einem molaren Verhältnis mit Methionin gleich wie oder höher als 2:1.

17. Verfahren gemäß Anspruch 16, **gekennzeichnet durch** die Verwendung einer oder mehr zusätzlicher Aminosäuren, deren Summe, ausgedrückt in Molekulargewicht, in einem Prozentsatz niedriger als 20 % in Bezug auf die Summe der anderen Wirkstoffe und weniger als 10 % für jede einzelne zusätzliche Aminosäure ist.

18. Verwendung der Aminosäuren Prolin, Glycin und Lysin in Kombination mit
- Leucin, Isoleucin und Threonin,
- Valin und
- einer oder mehr der anderen essentiellen Aminosäuren Phenylalanin, Histidin, Tryptophan und Methionin
als Wirkstoffe für die Herstellung einer Zusammensetzung zum Heilen und/oder Bessern von Wunden und Läsionen des Struktur- und Stromakollagens,
wobei Prolin, Glycin und Lysin in einer Gesamtmenge von bis zu 80 Gew.-% auf die Gesamtsumme aller ins Auge gefassten Aminosäuren oder Wirkstoffe vorliegt und Leucin, Isoleucin und Threonin in einem Gesamtquantum von zwischen 2 Gew.-% und 60 Gew.-% auf die Gesamtsumme aller ins Auge gefassten Aminosäuren oder Wirkstoffe vorliegt,
wobei die Mengen, ausgedrückt in Molekulargewicht, von Threonin und Lysin jeweils größer sind als die Summe der Einzelmengen der genannten anderen in der Zusammensetzung vorhandenen essentiellen Aminosäuren, aber:
- die Menge von Threonin kleiner ist als die Einzelmengen von Glycin, Prolin, Lysin, Leucin, Isoleucin und Valin;
- die Menge von Lysin kleiner ist als die Einzelmengen von Glycin, Prolin und Leucin.

19. Verwendung gemäß Anspruch 18, wobei die Summe der Mengen, ausgedrückt in Molekulargewicht, von Threonin und Lysin
- kleiner ist als die Summe der Einzelmengen von Glycin und Prolin und/oder
- kleiner ist als die Summe der Einzelmengen von Leucin, Isoleucin und Valin.

20. Verwendung gemäß Anspruch 18, wobei wenigstens eines von Methionin und Tyrosin als weiterer Wirkstoff vorgesehen ist.

21. Verwendung gemäß einem der vorhergehenden Ansprüche, worin Cyst(e)in, d. h. Cystin und Cystein, als weiterer Wirkstoff vorgesehen ist, vorzugsweise in einem molaren Verhältnis mit Methionin gleich wie oder höher als 2:1.

22. Verwendung gemäß Anspruch 21, worin eine oder mehr zusätzliche Aminosäuren vorgesehen sind, deren Summe, ausgedrückt in Molekulargewicht, in einem Prozentsatz niedriger als 20 % in Bezug auf die Summe der anderen Wirkstoffe und weniger als 10 % für jede einzelne zusätzliche Aminosäure ist.

## Revendications

1. Composition à base d'acides aminés, convenant en thérapie pour la guérison et/ou la réparation de plaies et de lésions, en particulier pour une application dans le domaine ophtalmique, comprenant :
- proline, glycine et lysine, en une quantité totale allant jusqu'à 80 % en poids du total de tous les acides aminés ou ingrédients actifs envisagés,
- leucine, isoleucine et thréonine, en une quantité totale comprise entre 2 % en poids et 60 % en poids du total de tous les acides aminés ou ingrédients actifs envisagés,
- valine
- un ou plusieurs des autres acides aminés essentiels phénylalanine, histidine, tryptophane et méthionine,
**caractérisée en ce que**
- les quantités, exprimées en poids moléculaire, de thréonine et de lysine sont chacune supérieures à la somme des quantités individuelles desdits autres acides aminés essentiels présents dans la composition, mais :
- la quantité de thréonine est inférieure aux quantités individuelles de glycine, proline, lysine, leucine, isoleucine et valine ;
- la quantité de lysine est inférieure aux quantités individuelles de glycine, proline et leucine.

2. Composition selon la revendication 1, **caractérisée en ce que** la somme des quantités, exprimées en poids moléculaire, de thréonine et de lysine
- est inférieure à la somme des quantités individuelles de glycine et de proline, et/ou
- est inférieure à la somme des quantités individuelles de leucine, isoleucine et valine.

3. Composition selon la revendication 1, **caractérisée en ce que** la somme des leucine, isoleucine valine et thréonine vaut jusqu'à 75 % en poids du total de tous les acides aminés ou ingrédients actifs envisagés.

4. Composition selon la revendication 1, **caractérisée en ce qu'**elle comprend au moins un parmi la méthionine et la tyrosine comme autre ingrédient actif.

5. Composition selon l'une quelconque des revendications précédentes, **caractérisée en ce qu'**elle comprend la cyst(é)ine, c'est-à-dire cystine et cystéine, comme autre ingrédient actif, de préférence en un rapport molaire avec la méthionine égal ou supérieur à 2:1.

6. Composition selon la revendication 5, **caractérisée en ce qu'**elle comprend un ou plusieurs acides aminés supplémentaires, dont la somme, exprimée en poids moléculaire, est, en pourcentage, inférieure à 20 % par rapport à la somme des autres ingrédients actifs et inférieure à 10 % pour chaque acide aminé supplémentaire individuel.

7. Composition selon au moins une des revendications précédentes **caractérisée en ce qu'**elle comprend :
- entre 8 % en poids et 40 % en poids de glycine sur le total des acides aminés envisagés ;
- entre 7 % en poids et 40 % en poids de proline sur le total des acides aminés envisagés ;
- entre 3 % en poids et 35 % en poids de glycine sur le total des acides aminés envisagés ;
la somme des glycine, proline et lysine n'étant, en particulier, pas inférieure à 18 % en poids du total des acides aminés envisagés.

8. Composition selon au moins une des revendications précédentes **caractérisée en ce qu'**elle comprend :
- entre 4 % en poids et 40 % en poids de leucine sur le total des acides aminés envisagés;
- entre 2 % en poids et 20 % en poids d'isoleucine sur le total des acides aminés envisagés;
- entre 2 % en poids et 20 % en poids de valine sur le total des acides aminés envisagés;
- jusqu'à 20 % pds de thréonine sur le total des acides aminés envisagés

9. Composition selon la revendication précédente, **caractérisée en ce que** la somme des leucine, isoleucine, valine et thréonine est comprise entre 10 % en poids et 50 % en poids sur le total des acides aminés envisagés.

10. Composition selon la revendication 8, **caractérisée en ce que** les leucine, isoleucine et valine sont en rapport stoechiométrique de 2:1:1.

11. Composition selon au moins une des revendications précédentes, **caractérisée en ce que** la thréonine plus la lysine sont en rapport molaire par rapport un autre acide aminé leucine, isoleucine et valine compris entre 20 % et 70 %, de préférence avec un rapport entre thréonine et lysine dans lequel la lysine est davantage représentée que la thréonine.

12. Composition selon au moins une des revendications précédentes **caractérisée en ce que** l'histidine est présente en un rapport molaire allant jusqu'à 50 % des acides aminés suivants :
- cyst(é)ine (c'est-à-dire cystine et cystéine) et méthionine ;
- phénylalanine et tyrosine ;
- tryptophane, en particulier en un rapport molaire allant jusqu'à 5 % du poids de tous les autres acides aminés sur une base de poids molaire.

13. Procédé de préparation d'une composition à base d'acides aminés, convenant en thérapie pour la guérison et/ou la réparation de plaies et de lésions, en particulier pour une application dans le domaine ophtalmique, faisant utilisation de :
- proline, glycine et lysine, en une proportion allant jusqu'à 80 % en poids du total de tous les acides aminés ou ingrédients actifs envisagés,
- leucine, isoleucine et thréonine, en une quantité totale comprise entre 2 % en poids et 60 % en poids du total de tous les acides aminés ou ingrédients actifs envisagés,
- valine,
- un ou plusieurs des autres acides aminés essentiels phénylalanine, histidine, tryptophane et méthionine,
**caractérisé en ce que**
- les quantités, exprimées en poids moléculaire, de thréonine et de lysine sont chacune supérieures à la somme des quantités individuelles desdits autres acides aminés essentiels présents dans la composition, mais :
- la quantité de thréonine est inférieure aux quantités individuelles de glycine, proline, lysine, leucine, isoleucine et valine ;
- la quantité de lysine est inférieure aux quantités individuelles de glycine, proline et leucine.

14. Procédé selon la revendication 13, **caractérisé en ce que** la somme des quantités, exprimées en poids moléculaire, de thréonine et de lysine
- est inférieure à la somme des quantités individuelles de glycine et de proline, et/ou
- est inférieure à la somme des quantités individuelles de leucine, isoleucine et valine.

15. Procédé selon la revendication 13, **caractérisé par** l'utilisation d'au moins un parmi la méthionine et la tyrosine comme autre ingrédient actif.

16. Procédé selon l'une quelconque des revendications précédentes, **caractérisé par** l'utilisation de cyst(é)ine, c'est-à-dire cystine et cystéine, comme autre ingrédient actif, de préférence en un rapport molaire avec la méthionine égal ou supérieur à 2:1.

17. Procédé selon la revendication 16, **caractérisé par** l'utilisation d'un ou plusieurs acides aminés supplémentaires, dont la somme, exprimée en poids moléculaire, est, en pourcentage, inférieure à 20 % par rapport à la somme des autres ingrédients actifs et inférieure à 10 % pour chaque acide aminé individuel.

18. Utilisation des acides aminés proline, glycine et lysine en combinaison avec
- leucine, isoleucine et thréonine,
- valine, et
- un ou plusieurs des autres acides aminés essentiels phénylalanine, histidine, tryptophane et méthionine,
comme ingrédients actifs pour la fabrication d'une composition pour la guérison et/ou la réparation de plaies et de lésions du collagène structural et stromal,
les proline, glycine et lysine étant en une quantité totale allant jusqu'à 80 % en poids du total de tous les acides aminés ou ingrédients actifs envisagés, et les leucine, isoleucine et thréonine, étant en une quantité totale comprise entre 2 % en poids et 60 % en poids du total de tous les acides aminés ou ingrédients actifs envisagés,
dans laquelle les quantités, exprimées en poids moléculaire, de thréonine et de lysine sont chacune supérieures à la somme des quantités individuelles desdits autres acides aminés essentiels présents dans la composition, mais :
- la quantité de thréonine est inférieure aux quantités individuelles de glycine, proline, lysine, leucine, isoleucine et valine ;
- la quantité de lysine est inférieure aux quantités individuelles de glycine, proline et leucine.

19. Utilisation selon la revendication 18, dans laquelle la somme des quantités, exprimées en poids moléculaire, de thréonine et de lysine
- est inférieure à la somme des quantités individuelles de glycine et de proline, et/ou
- est inférieure à la somme des quantités individuelles de leucine, isoleucine et valine.

20. Utilisation selon la revendication 18, dans laquelle au moins une parmi la méthionine et la tyrosine est fournie comme autre ingrédient actif.

21. Utilisation selon l'une quelconque des revendications précédentes, dans laquelle la cyst(é)ine, c'est-à-dire cystine et cystéine, est fournie comme autre ingrédient actif, de préférence en un rapport molaire avec la méthionine égal ou supérieur à 2:1.

22. Utilisation selon la revendication 21, dans laquelle un ou plusieurs acides aminés supplémentaires sont fournis, dont la somme, exprimée en poids moléculaire, est, en pourcentage, inférieure à 20 % par rapport à la somme des autres ingrédients actifs et inférieure à 10 % pour chacun des acides aminés individuels supplémentaires.
